(19) **Europäisches Patentamt**

**European Patent Office**

**Office européen des brevets**

(11) **EP 0 721 512 B1**

(12) **EUROPEAN PATENT SPECIFICATION**

(45) Date of publication and mention
of the grant of the patent:
**08.11.2000 Bulletin 2000/45**

(51) Int. Cl.[7]: **C12Q 1/00**, C12Q 1/02,
C12Q 1/26, G01N 27/409

(21) Application number: **94922387.9**

(22) Date of filing: **01.08.1994**

(86) International application number:
**PCT/KR94/00103**

(87) International publication number:
**WO 96/04399 (15.02.1996 Gazette 1996/08)**

(54) **QUICK BIOCHEMICAL OXYGEN DEMAND TEST AND APPARATUS FOR THE SAME**

SCHNELLER TEST DES BIOCHEMISCHEN SAUERSTOFFBEDARFES UND GERÄT DAFÜR

TEST DE DEMANDE BIOCHIMIQUE D'OXYGENE RAPIDE ET APPAREIL POUR EFFECTUER CE TEST

(84) Designated Contracting States:
**DE FR GB IT**

(43) Date of publication of application:
**17.07.1996 Bulletin 1996/29**

(73) Proprietor: **YUKONG LIMITED
Yongdungpo-gu, Seoul 150-010 (KR)**

(72) Inventors:
• **PARK, Yong, Seck
Suh-gu Taejon 302-173 (KR)**
• **KIM, Hyung, Charn
Taejon 300-090 (KR)**
• **KIM, Sung, Hong
Samsung Pureun
Taejon 305-390 (KR)**
• **YI, Yong, Taek
Taejon 302-172 (KR)**

(74) Representative:
**Lins, Edgar, Dipl.-Phys. Dr.jur. et al
GRAMM, LINS & PARTNER
Theodor-Heuss-Strasse 1
38122 Braunschweig (DE)**

(56) References cited:
**EP-A- 0 369 490**      **DE-A- 4 301 087**
**US-A- 4 350 763**

• **PATENT ABSTRACTS OF JAPAN, unexamined applications, section P, Vol. 15, No. 402, issued 14 October 1991, The Patent Office Japanese Government, page 163, P 1262; & JP,A,3 163 350 (KAO CORP.), 15-07-91.**
• **PATENT ABSTRACTS OF JAPAN, unexamined applications, section P, Vol. 14, No. 500, issued 31 October 1990, The Patent Office Japanese Government, page 139, P 1125; & JP,A,2 206 761 (KYORITSU YUKI CO LTD.), 16-08-90.**
• **PATENT ABSTRACTS OF JAPAN, unexamined applications, section P, Vol. 13 No. 253, issued 13 June 1989, The Patent Office Japanese Government, page 72, P 883; & JP,A,1 050 947 (FUJI ELECTRIC CO LTD.), 27-02-89.**
• **PATENT ABSTRACTS OF JAPAN, unexamined applications, section P, Vol. 7 No. 18, issued 25 January 1983, The Patent Office Japanese Government, page 102, P 170; & JP,A,57 173 745 (MITSUBISHI DENKI K.K.), 26-10-82.**

**Description**

BACKGROUND OF THE INVENTION

Field of the Invention

The present invention relates in general to a novel method for the determination of biochemical oxygen demand in aqueous liquids containing organic matter and an apparatus for the same, and more particularly to improvements in quickness and accuracy with the method and the apparatus.

Description of the Prior Art

Generally, biochemical oxygen demand (hereinafter "BOD") of an aqueous liquid contaminated with organic matter, which is defined as an amount of dissolved oxygen necessary to the metabolic activity of the microorganisms that oxidize the biologically decomposable organic matter to grow, is used as an index that reflects the state of water contamination. BOD is of great importance in industrial working spots. For example, when carrying out a biological process for waste water treatment, the BOD values of influent and in aeration basin are important parameters in operating the process normally and in preventing a variety of problems. In addition, since the BOD of effluent is regulated below a legal limit, a manager always measures the value of BOD to control the effluent quality.

While several quick methods were suggested for the determination of BOD, it is still common practice to measure BOD by Standard Method No. 219 of the American Public Health Association or by the closely similar method of Japanese Industrial Standard JIS K0102-1974. Both methods, however, require a test solution to be held under prescribed conditions for five days before the result of the test can be known. Particularly, the five days is based on the fact that it takes a long time for aerobic microorganisms to grow after a sample is added into a dilute aqueous liquid saturated with oxygen, and thus, BOD measured by the conventional method, in which five days is required to perform the test, is represented as $BOD_5$. What is worse, the conventional method includes another problem such that the value of the test results is in dependence on the skill of the operator, because it is very complicated and intricate. Hence, the above conventional methods are not convenient to control effluent from industrial plants and the like, which requires quickness and accuracy.

One prior art proposal for reducing the test period to, for example, 30 minutes, was disclosed in U.S. Patent No. 4,350,763 by Shuichi Suzuki at el. issued on 1982 (hereinafter "763' patent"). This method suggests that a sample solution comes into contact with molecular oxygen and with immobilized microorganisms capable of aerobically metabolizing organic matter in an aqueous liquid and of thereby consuming the oxygen. In the 763' patent, an adequate number of microorganisms are immobilized to establish the oxygen-consuming ability of the immobilized cells when in contact with solutions having known BOD and to compare the rate of oxygen consumption of the same microorganisms in contact with an unknown test sample with a calibration chart derived from the tests on known standard samples. That is, the 763' patent makes use of such a principle that the rate of oxygen consumption is directly proportional to the BOD, so that a plot of BOD vs rate of oxygen consumption is represented by a straight line in a system of Cartesian coordinates. For example, microorganisms which are immobilized in membrane connected with an electrode come into contact with oxygen-saturated buffer free of oxidizable organic matter, in order to establish a constant current indicative of a reference dissolved oxygen (hereinafter "DO"). And when the oxygen-sensitive electrode is immersed in waste water or the like which contains molecular oxygen, the oxygen content of this sample solution is sensed by the electrode. As the immobilized microorganisms metabolize the organic material in the waste water, the resulting variation in the output current of the electrode is obtained. The method utilizing the oxygen-sensitive electrode proposed in 763' patent is advantageous in many aspects, such as quickness, simplicity, and accuracy, as compared with the conventional $BOD_5$.

However, the method of 763' patent has such disadvantages as follow: first, if the number of the microorganisms immobilized in membrane is not constant, the rates of oxygen consumption are different in respective test case for even the same waste water and thence, wrong results of BOD are obtained; second, a standard curve is necessary to plot a calibration chart, which results from indirect determination of BOD, so that another standard curve is drawn again, depending on the activity variation of the microorganisms; third, if some constituents of the organic matter do not pass through a general bio-degradation pathway, BOD values thereof are impossible to measure or may be lower than the true values.

SUMMARY OF THE INVENTION

For solving the aforementioned problems, the inventors have recognized that there exists a need for a faster test less sensitive to human error, dispensable from standard curve, and indifferent to bio-degradation pathway of organic matter.

Accordingly, in an aspect of the present invention, there is provided a method for determining BOD of aqueous liquids containing organic matter, whereby a BOD result can be obtained much faster, normally within 20 minutes.

According to another aspect of the present invention, there is provided a method for determining BOD, less sensitive to the operator's skill.

According to a further aspect of the present invention, there is provided a method for determining BOD unnecessary of any standard curve. According yet another aspect of the present invention, there is provided a method for determining BOD, applicable to any aqueous liquid containing the organic matter under unusual bio-degradation pathway.

According to a concomitant aspect of the present invention, there is provided an apparatus for performing the method.

In accordance with the present invention, the above objects can be accomplished by providing a method for determining BOD in an aqueous liquid containing organic matter, comprising the steps of aerating a culture of microorganisms to exhaust the organic matter in the culture until an exhausted state is achieved; introducing said aerated culture into a quick biochemical oxygen demand (QBOD) bottle; feeding a sample containing the organic matter degradable by the microorganisms into said QBOD bottle, to measure an amount of oxygen consumed by the microoranisms; calculating the BOD of the sample.

The above objects can be also achieved by providing an apparatus for determining the BOD of an aqueous liquid containing organic matter by the above method, which comprises a magnetic stirrer, a quick biochemical oxygen demand (QBOD) bottle provided with a hole for a dissolved oxygen (DO)/temperature sensor connected to a recording instrument and with a protruded feed hole, an aerating instrument providing air to a microorganism culture tank through an aeration stone, a tubing pump transferring the microorganism culture from the microorganism culture tank to the QBOD bottle through an inlet hole formed at the lower portion of the QBOD bottle and another tubing pump draining the microorganism culture from the QBOD bottle through an outlet hole formed at the oppocite lower portion of the QBOD bottle.

BRIEF DESCRIPTION OF THE DRAWINGS

The above and other object, features and advantages of the present invention will be more apparent from the following detailed description taken with reference to the accompanying drawings, in which:

FIG. 1 is a flow sheet of an apparatus suitable for performing the method of the present invention;
FIG. 2 is a graph illustrating the profile of DO decrease according to Example 1 of the present invention;
FIG. 3 is a graph illustrating the DO decrements according to Example 1 of the present invention;
FIG. 4 is a graph illustrating the QBOD and $BOD_5$ with regard to the concentration of methanol according to Example 1;
FIG. 5 is a graph illustrating the DO decrements depending on the addition of acetic acid, ethanol and phenol solutions according to Example 2 of the present invention; and
FIG. 6 is a graph illustrating the stability of the microorganism culture according to Example 2 of the present invention.

DETAILED DESCRIPTION OF THE INVENTION

The preferred embodiment of the present invention will be, in detail, described with reference to the accompanying drawings.

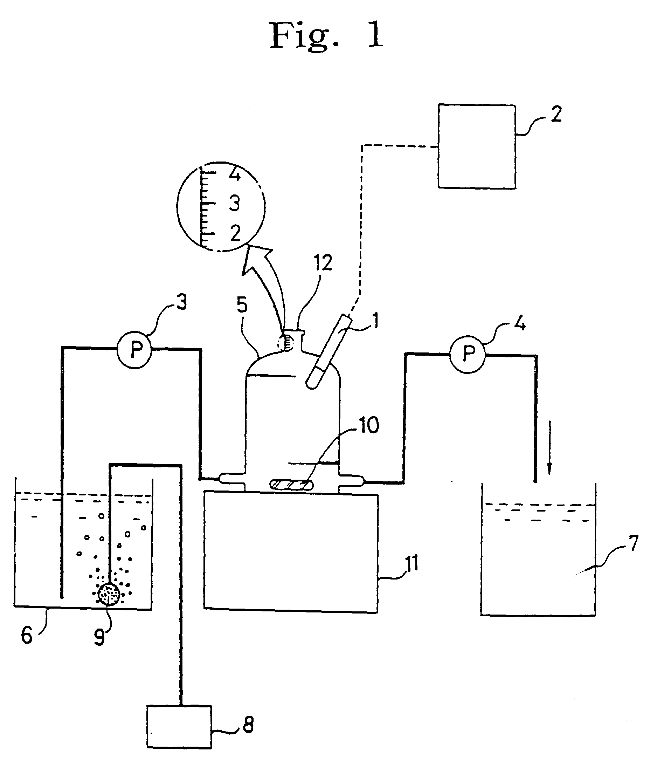

Referring to FIG. 1, there is shown an apparatus comprising of a QBOD bottle 5 having a hole in which a DO/temperature sensor 1 connected to a recording instrument 2 is obliquely installed, two tubing pumps 3 and 4 connected with the QBOD bottle so as to deliver microorganism culture from microorganism culture tank 6 and a container 7, an aerating instrument 8 providing air through an aeration stone 9 to the tank 6, and a magnetic stirrer 11 supporting the QBOD bottle 5 and operating a magnetic bar 10 in the bottle. To operate the QBOD system illustrated in FIG. 1, a microorganism culture such as a solution of an aeration basin, a return sludge and an artificial culture are contained in the tank 6 which is then aerated through the aeration stone by the aerating instrument 8. The organic matter which is usable to microorganisms in the culture is exhausted so completely as to keep a DO value high. That is, air is provided to exhaust the soluble organic matter in the culture until oxygen uptake rate is not more than 20 $ppm-O_2/h$. The resulting culture is introduced into the QBOD bottle 5 by a tubing pump 3 in such a way to fill upto a bottle neck which is notched into a scale and, thereafter, is stirred with the magnetic bar 10 operating in a constant rate by means of the magnetic stirrer 11, so that DO is consumed in a very slow rate. When a predetermined amount of sample solution containing the organic matter is fed to the QBOD bottle through the feed bole 12, the oxygen is consumed as rapid as the BOD of the fed organic matter for a few minutes and then, the consumption rate shows the inherited slow rate. In the above procedure, the total amount of the consumed oxygen is the amount consumed by the microorganisms themselves and the consumption amount based on the addition of the organic matter. Therefore, the total amount of the consumed oxygen minus the amount consumed by the microorganisms themselves leaves BOD of the organic matter. With regard to a BOD range, from 10 to 10,000 ppm of BOD is measurable without diluting a sample.

As illustrated above, according to the method of the present invention, BOD of a sample is capable of being determined directly by exhausting the organic matter of microorganism culture, adding organic matter to the exhaust culture and measuring the consumed oxygen. Accordingly, the number of the microorganisms has influence on only the measuring time but does not affect the BOD results. In accordance with the present invention, BOD is determined directly in total, so that any standard curve is not necessary. In addition, there is another advantage in the method, in that a measurable range of BOD is from 10 to 10,000 ppm, unknown sample is not to be diluted. Furthermore, in case that the method according to the present invention is applied to waste water treatment plant, since the microorganisms for the test are those that are generated from the plant and thus, adapted to the constituents of the waste water, the BOD values of all aqueous liquids from influent for treatment, a solution in an aeration basin and the like are determined accurately.

As compared with the conventional $BOD_5$, the method according to the present invention has such advantages as follow: 1st, it is much faster (from 5 days to 20 minutes); 2nd, it is better reproducible, being less dependent on operator's skill; 3rd, it is unnecessary to keep a special temperature such as 20 °C for the sample; 4th, it is much more accurate for the BOD test measurement for standard solution; 5th, it is not necessary to inoculate microorganisms; 6th, it is not affected by nitrifying bacteria; 7th, it is not largely affected, even if the dilute water is contaminated with microorganisms; 8th, it has a measurable BOD range of about 10 to 10,000 ppm, so that repetition of test or dilution is not necessary.

In an apparatus for performing the QBOD test according to the present invention, as shown in FIG. 1, a 300 mL transparent QBOD bottle 5 has a sensor hole for DO/temperature sensor 1 and a protrude feed hole calibrated at upper portions and has, at both opposite sides of lower portion, two protrusions which are connected with a microorganism culture tank 6 and a container 7 through tubing pumps 3 and 4, respectively. Any commercially available DO/temperature sensor may be used in the present invention. For example, the DO/temperature sensors manufactured and sold by YSI company, USA or Horiba company, Japan may be used. The DO/temperature sensor senses the DO value of test sample. The sensor is installed obliquely as shown in FIG. 1 to remove test errors generated by bubbles. The DO/temperature sensor is connected to a recording instrument 2, which records a signal generated by the sensor in response to the DO value and which calculates the consumed amount of oxygen with a programmed calculation method and a timer (not shown), so as to determine BOD by itself. The QBOD bottle 5 is mounted on a magnetic stirrer 11 and includes a magnetic bar 10 which homogeneously stirs a solution to be tested. The recording instrument 2 which receives the signals of DO values may print decrements with regard to time or plot a graph thereabout. The microorganism culture in the tank 6 is saturated with air which is delivered by an air pump 8 through an aeration stone. Through the tubing pumps 3 and 4 which are connected to the QBOD bottle 5, the saturated culture is introduced to the bottle 5 and the used culture is discharged from the bottle 5. Besides operating the magnetic bar 10, the magnetic stirrer 11 may memory the amount of a sample to calculate a dilution, which is subsequently utilized for the calculation of BOD. For example, if 0.5 mL of the sample is inputted into the magnetic stirrer 11, the dilution is calculated to 600 (300/0.5), which is automatically utilized for the calculation of BOD with an operating chip equipped.

EXAMPLE 1

About 1 L of the waste water gathered from aeration basin of the waste water treatment spot of a petrochemical plant was poured into the tank 6 at 25 °C and then, the tank was saturated with air through an aeration stone till the DO value of the waste water is constant. When the DO value was constant in the state of above 80 %, the aerated waste water was introduced into the QBOD bottle 5 so as to fill up to the neck thereof and stirred in a medium rate of 600 to 700 rpm by 2,14 cm (1 inch) bar. From the point when the DO value was decreased by the microorganisms suspended in the waste water, the values were recorded every 30 seconds. Methanol solutions of 10, 25, 50, 75, 100, 250, 500, 750 and 1,000 ppm were prepared using methanol of reagent grade.

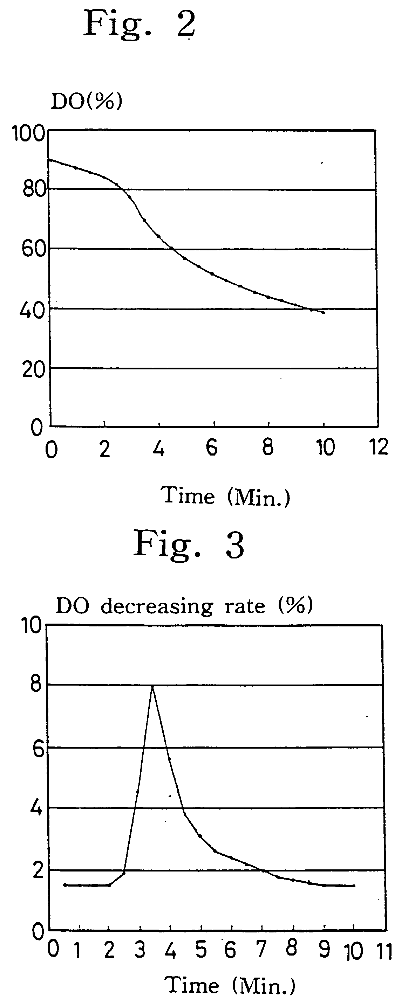

When the oxygen uptake rate (OUR) of microorganisms was constant, 0.5 mL of the methanol solution of 1,000 ppm was fed, through a feed hole, into the QBOD bottle and DO values and DO decrements were, then, measured. The results are given as shown in Table 1 and illustrated in FIGS. 2 and 3.

After feeding the sample, the OUR increased for 8 minutes and returned to the inherited value. At that time, DO value was 41.3 %. During this period, the total variation of DO value was 45.7 %, which include the inherited amount, 24 % (3.0 % x 8 min.) of oxygen consumed by the microorganisms. As a result, a net amount of oxygen consumption according to the addition of the sample became 21.7 %. Therefore, the BOD of the sample is calculated as follows:

$$\text{Sample BOD} = 21.7\ \%\ \times\ \left(\frac{8.3\ \text{ppm}}{100\ \%}\right)\ \times\ \left(\frac{300\ \text{ml}}{0.5\ \text{ml}}\right) = 1{,}081\ \text{ppm}$$

wherein 8.3 ppm is the concentration when oxygen is saturated in water at 25 °C.

The DO values and DO decrements were printed in the QBOD apparatus according to the present invention, and the DO decrements with regard to time were shown in FIG. 3. In FIG. 3, while both ends of curve shaping a bell means that the inherited OUR by the microorganisms is 1.5 % per 30 seconds, the curve shaping the bell means that the OUR is changed depending on the concentration of the organic matter.

The culture used in the QBOD bottle 5 was released into the container 7 and fresh culture in tank 6 was introduced into the bottle 5 because a high DO value was required to determine the BOD of next sample. During the determination of BOD, the microorganism culture in the container 7 was poured into the tank 6 and then aerated. The solutions of 100, 250, 500 and 1,000 ppm were tested in a manner similar to the aforementioned that, and the results were 112, 280, 525 and 803 ppm, respectively. 5 ml of the methanol solutions of 10, 25, 50, 75 and 100 ppm were also tested in a manner similar to the aforementioned manner and the results were 11, 27, 59, and 104 ppm, respectively.

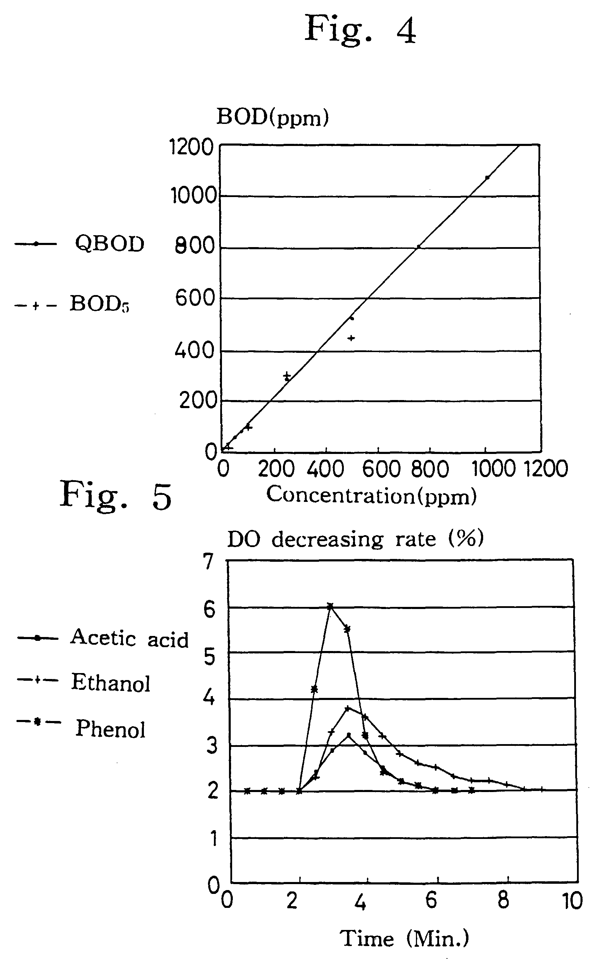

With regard to the methanol solutions of 25, 100, 250 and 500 ppm, $BOD_5$ values were measured, using Model 2173B of HACH company, USA., a manometer-type BOD measuring apparatus. The results were 20, 115, 290 and 450 ppm, respectively. QBOD and $BOD_5$ are plotted in FIG. 4.

EXAMPLE 2

About 1 L of the waste water gathered from aeration basin of the waste water treatment spot of a petrochemical plant was poured into the tank 6 at 25 °C and then, the tank was saturated with air through an aeration stone till the DO value of the waste water is constant. When the DO value was constant in a state of above 80 %, the aerated waste water was introduced into the QBOD bottle 5 so as to fill up to the neck thereof and stirred in a medium rate of 600 to 700 rpm by 1 inch bar. From the point when the DO value was decreased by the microorganisms suspended in the waste water, the values were recorded every 30 seconds. Using acetic acid, ethanol and phenol which were graded into a reagent, each solution of 500 ppm were prepared.

When the OUR by the microorganisms was constant in the QBOD bottle, 0.5 mL of the acetic acid solution was fed through a feed hole into the QBOD bottle and then "0.5 mL" was inputted into the magnetic stirrer. In 10 minutes after feeding the sample, BOD value were shown at a display window of the recording instrument. With regard to the ethanol and the phenol solutions, the test results were 438 and 478 ppm, respectively. The DO decrements were printed every 30 seconds in the QBOD apparatus according to the present invention, and were shown in FIG. 5.

$BOD_5$ values were measured for the above three solutions and the results were 220, 445 and 505 ppm, respectively.

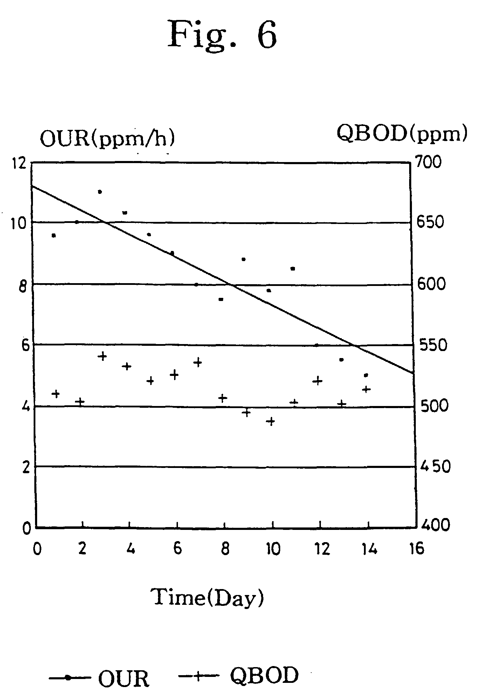

To test the stability of the microorganism culture with regard to time, the inherited OUR and the QBOD of methanol of 500 ppm were measured. The inherited OUR of the microorganism culture which was left at 25 °C for a time was inclined to decrease with regard to time, as shown in FIG. 6. However, the QBOD value was constant for over 15 days, being indifferent to the inherited OUR. During 15 days, an average value of QBOD was 514 ppm and the standard deviation was 16.4. When the BOD of sample was measured with the microorganism culture left, the aeration in the tank 6 was operated for not less than 1 hour so as to keep the DO value constant in a state of not less than 80 %.

TABLE 1

| DO variations depending on the addition of MeOH solution | | | | | | | |
|---|---|---|---|---|---|---|---|
| Sample : MeOH 1,000 ppm | | | | | | | |
| Time (min) | DO (%) | ΔDO (%) | Remark | Time (min) | DO (%) | ΔDO (%) | Remark |
| 0 | 90 | | 25.0°C | 5.5 | 54.6 | 2.6 | |
| 0.5 | 88.5 | 1.5 | | 6 | 52.1 | 2.4 | |
| 1 | 87.0 | 1.5 | sample add | 6.5 | 49.9 | 2.2 | |
| 1.5 | 85.5 | 1.5 | | 7 | 47.9 | 2.0 | |
| 2 | 84.0 | 1.5 | | 7.5 | 46.1 | 1.8 | |
| 2.5 | 82.1 | 1.9 | | 8 | 44.4 | 1.7 | |

TABLE 1 (continued)

| DO variations depending on the addition of MeOH solution | | | | | | | |
|---|---|---|---|---|---|---|---|
| Sample : MeOH 1,000 ppm | | | | | | | |
| 3 | 77.6 | 4.5 | | 8.5 | 42.8 | 1.6 | |
| 3.5 | 69.6 | 8.0 | | 9 | 41.3 | 1.5 | finish |
| 4 | 64.0 | 5.6 | | 9.5 | 39.8 | 1.5 | |
| 4.5 | 60.0 | 3.8 | | 10 | 38.3 | 1.5 | 25.1°C |
| 5 | 57.4 | 3.1 | | | | | |

**Claims**

1. A method for determining the biochemical oxygen demand (BOD) of an aqueous liquid containing organic matter using a culture of microorganisms, which comprises the steps of:

   aerating a culture of microorganisms to exhaust the organic matter in the culture until an exhausted state is achieved;

   introducing said aerated culture into a quick biochemical oxygen demand (QBOD) bottle;

   feeding a sample containing the organic matter degradable by the microorganisms into said QBOD bottle, to measure an amount of oxygen consumed by the microoranisms; and

   calculating the BOD of the sample.

2. The method according to claim 1, in which the step of aerating the culture is continued until the oxygen uptake rate of the microorganisms is not more than 20 ppm-$O_2$/h.

3. The method according to claim 1 or 2, in which the microorganism culture is selected from the group consisting of a solution from an aerated basin, a return sludge and an artificial microorganism culture.

4. The method according to one of the claims 1 to 3, in which the BOD value is calculated from only the amount of the consumed oxygen and a dilution, without using a standard curve.

5. The method according to one of the claims 1 to 4, in which the BOD of the sample is measured in a range of about 10 to about 1,000 ppm, without diluting.

6. A method according to one of the claims 1 to 5, in which the BOD is determined within 20 minutes after the step of sample adding.

7. An apparatus for determining the biochemical oxygen demand (BOD) of an aqueous liquid containing organic matter by the method according to one of the claims 1 to 6, comprising a magnetic stirrer, a quick biochemical oxygen demand (QBOD) bottle provided with a hole for a dissolved oxygen (DO)/temperature sensor connected to a recording instrument and with a protruded feed hole, an aerating instrument providing air to a microorganism culture tank through an aeration stone, a tubing pump transferring the microorganism culture from the microorganism culture tank to the QBOD bottle through an inlet hole formed at the lower portion of the QBOD bottle and another tubing pump draining the microorganism culture from the QBOD bottle through an outlet hole formed at the opposite lower portion of the QBOD bottle.

8. The apparatus according to claim 7 in which the hole for the DO/temperature sensor is formed obliquely at the upper portion of the QBOD bottle and the protruded feed hole is calibrated

**Patentansprüche**

1. Verfahren zur Bestimmung des biochemischen Sauerstoffbedarfs (BOD) einer organisches Material enthaltenden wässrigen Flüssigkeit unter Verwendung einer Mikroorganismenkultur, mit den Verfahrensschritten:

   Belüftung einer Mikroorganismenkultur zum Verbrauchen des organischen Materials bis ein erschöpfter Zustand erreicht wird;

   Einbringen der belüfteten Kultur in eine Flasche (QBOD) zur Schnellbestimmung des biochemischen Sauerstoffbedarfs;

   Zuführen einer das von den Mikroorganismen abbaubare organische Material enthaltenden Probe zu der QBOD-Flasche zur Messung einer von den Mikroorganismen verbrauchten Sauerstoffmenge; und

   Berechnung des BOD der Probe.

2. Verfahren nach Anspruch 1, bei dem der Schritt der Belüftung der Kultur fortgeführt wird, bis die Sauerstoffaufnahmerate des Mikroorganismus nicht höher als 20 ppm-$O_2$/h ist.

3. Verfahren nach Anspruch 1 oder 2, bei dem die Mikroorganismenkultur aus der Gruppe ausgewählt ist, die aus einer Lösung aus einem belüfteten Behälter, einem Rücklaufschlamm und einer künstlichen Mikroorganismenkultur gebildet wird.

4. Verfahren nach einem der Ansprüche 1 bis 3, bei dem der BOD-Wert nur aus der Menge des verbrauchten Sauerstoffs und einer Verdünnung ohne Benutzung einer Normierungskurve berechnet wird.

5. Verfahren nach einem der Ansprüche 1 bis 4, bei dem der BOD der Probe in einem Bereich von 10 bis etwa 1.000 ppm ohne Verdünnung gemessen wird.

6. Verfahren nach einem der Ansprüche 1 bis 5, bei dem der BOD innerhalb von 20 Minuten nach dem Schritt der Zugabe der Probe bestimmt wird.

7. Gerät zur Bestimmung des biochemischen Sauerstoffbedarf (BOD) einer organischen Material enthaltenden wässrigen Flüssigkeit nach dem Verfahren nach einem der Ansprüche 1 bis 6, mit einem magnetischen Rührer, einer Flasche (QBOD) zur Schnellbestimmung des biochemischen Sauerstoffbedarfs, die mit einem Loch für einen mit einem Aufzeichnungsinstrument verbundenen Sensor für gelösten Sauerstoff (DO)/Temperatur und mit einer vorstehenden Zuführungsöffnung versehen ist, einem Belüftungsinstrument, das einen Mikroorganismenkulturtank über einen Belüftungsstein mit Luft versorgt, einer in eine Leitung eingesetzten Pumpe zum transportieren der Mikroorganismenkultur von dem Mikroorganismenkulturtank zur QBOD-Flasche durch eine im unteren Bereich der QBOD-Flasche angebrachte Einlassöffnung und einer weiteren in eine Rohrleitung eingesetzten Pumpe zum Abziehen der Mikroorganismenkultur aus der QBOD-Flasche durch eine im unteren Abschnitt der QBOD-Flasche gegenüber ausgebildeten Auslassöffnung.

8. Gerät nach Anspruch 7, bei dem das Loch für den DO/Temperatursensor im oberen Abschnitt der QBOD-Flasche schräg ausgebildet und die vorstehende Einführungsöffnung kalibriert ist.

**Revendications**

1. Un procédé pour déterminer la demande en oxygène biochimique (BOD) d'un liquide aqueux renfermant une substance organique en utilisant une culture de micro-organismes qui comprend les étapes consistant à :

   aérer une culture de micro-organismes pour évacuer la substance organique de la culture jusqu'à l'obtention d'un état d' évacuation ;
   introduire ladite culture aérée dans une bouteille de demande d'oxygène biochimique rapide (QBOD) ;
   amener un échantillon renfermant la substance organique apte à être dégradée par les micro-organismes dans ladite bouteille QBOD pour mesurer une quantité d'oxygène consommé par les micro-organismes ; et calculer la BOD de l'échantillon.

2. Le procédé selon la revendication 1, dans lequel l'étape d'aération de la culture est poursuivie jusqu'à ce que la vitesse d'absorption d'oxygène des micro-organismes ne soit pas supérieure à 20 ppm-$O_2$/h.

3. Le procédé selon la revendication 1 ou 2, dans lequel la culture de micro-organismes est choisie parmi le groupe comprenant une solution provenant d'un réceptacle aéré, une bouillie de retour et une culture de micro-organismes artificielle.

4. Le procédé selon l'une des revendications 1 à 3, dans lequel la valeur BOD est calculée seulement à partir de la quantité de l'oxygène consommé et d'une dilution, sans utiliser une courbe standard.

5. Le procédé selon l'une des revendications 1 à 4, dans lequel la BOD de l'échantillon est mesurée dans une gamme d'environ 10 à environ 1000 ppm, sans dilution.

6. Un procédé selon l'une des revendication 1 à 5, dans lequel la BOD est déterminée en 20 minutes après l'étape d'addition d'échantillon.

7. Un appareil pour déterminer la demande d'oxygène biochimique (BOD) d'un liquide aqueux renfermant une substance organique par le procédé selon l'une des revendications 1 à 6, comprenant un agitateur magnétique, une bouteille de demande d'oxygène biochimique rapide (QBOD) avec un trou pour un capteur de température/oxygène dissous (DO) relié à un instrument d'enregistrement et avec un trou d'alimentation faisant saillie, un instrument d'aération fournissant de l'air à un récipient de culture de micro-organismes par l'intermédiaire d'un moyen d'aération, une pompe à tubage transférant la culture de micro-organismes depuis le récipient de culture de micro-organismes à la bouteille QBOD par l'intermédiaire d'un trou d'entrée ménagé dans la partie inférieure de la bouteille QBOD et une autre pompe à tubage drainant la culture de micro-organismes de la bouteille QBOD par l'intermédiaire d'un trou de sortie formé sur la partie inférieure opposée de la bouteille QBOD.

8. L'appareil selon la revendication 7, dans lequel le trou pour le capteur de température/DO est formé obliquement dans la partie supérieure de la bouteille QBOD et le trou d'alimentation faisant saillie est calibré.

EP 0 721 512 B1

# Fig. 1

9

# Fig. 2

DO(%)

Time (Min.)

# Fig. 3

DO decreasing rate (%)

Time (Min.)

# Fig. 4

BOD(ppm)

—•— QBOD

—+— BOD₅

# Fig. 5

DO decreasing rate (%)

—•— Acetic acid

—+— Ethanol

—*— Phenol

Time (Min.)

# Fig. 6

OUR(ppm/h)　　　QBOD(ppm)

Time(Day)

—•— OUR　—+— QBOD